# EUROPÄISCHE PATENTSCHRIFT

(11) **EP 2 285 316 B1**
(45) Veröffentlichungstag und Bekanntmachung des Hinweises auf die Patenterteilung: **06.06.2012**
(21) Anmeldenummer: 09727127.4
(22) Anmeldetag: 02.04.2009
(51) Int. Cl.: A61F 2/80

(54) **GLIEDMASSENSTUMPFAUFNAHMEHÜLSE MIT INTEGRIERBARER ARRETIER-VORRICHTUNG FÜR EIN DICHTELEMENT**
LIMB STUMP RECEIVING SLEEVE COMPRISING AN INTEGRATED LOCKING DEVICE FOR A SEALING ELEMENT
MANCHON DE RÉCEPTION DE MOIGNON POURVU D'UN DISPOSITIF DE BLOCAGE INTÉGRABLE POUR UN ÉLÉMENT D'ÉTANCHÉITÉ

(30) Priorität: 05.04.2008 DE 202008004714 U; 26.04.2008 DE 102008021054
(43) Veröffentlichungstag der Anmeldung: 23.02.2011
(73) Patentinhaber: medi GmbH & Co. KG, 95448 Bayreuth (DE)
(72) Erfinder: KURTH, Christof, 95500 Heinersreuth (DE)
(74) Vertreter: Blaumeier, Jörg
(86) Internationale Anmeldenummer: PCT/DE2009/000425
(87) Internationale Veröffentlichungsnummer: WO 2009/121340

(56) Entgegenhaltungen:
- EP-A- 0 631 765
- EP-A- 1 875 882
- WO-A-03/024370
- DE-A1- 2 060 239

## Beschreibung

Die vorliegende Erfindung betrifft eine Gliedmaßenstumpfaufnahmehülse mit integrierbarer oder integrierter Aufnahme für ein Dichtungselement mit welchem ein Gliedmaßenstumpf in einer Stumpfaufnahmehülse mittels Abdichtung des distal befindlichen Bereiches des Inneren der Stumpfaufnahmehülse zum Gliedmaßenstumpf und der umgebenden Atmosphäre hin, arretiert wird. Das Dokument EP-A-1 875 882 stellt der nächste Stand der Technik dar.

Bei der Verbindung des Stumpfes eines Patienten mit einer Gliedmaßenstumpfaufnahmehülse wird üblicher Weise der mit einem Liner überzogene Stumpf von oben, in Richtung des geschlossenen, distalen Endes der Stumpfaufnahmehülse eingebracht und fixiert.

Die Fixierung erfolgt durch einen erzeugten Unterdruck in der Gliedma-βenstumpfaufnahmehülse beim Einbringen des Stumpfes, wofür zahlreiche Stumpfaufnahmehülsen nahe ihrem distalen Ende über ein Ventil verfügen, über welches die komprimierte Luft während des Eintauchens des Stumpfes aus der Hülse entweichen kann.

Die Abdichtung des Liners gegenüber der Aufnahmehülse erfolgt bei vielen Ausführungsformen nur durch Abdichtung in Folge flächigen Anliegens zwischen diesen beiden Teilen selbst und ist abhängig von den verwendeten Materialien und der maßlichen Ausführung des Liners und des Inneren der Stumpfaufnahmehülse. Wenn der Lineraußendurchmesser mindestens dem Schaftinnendurchmesser entspricht, kann über diese Form der flächigen Abdichtung Unterdruck im Schaft dauerhaft erzeugt werden. Ist der Außendurchmesser des Gliedmaßenstumpfs inkl. Liner jedoch größer als der Schaftinnendurchmesser wird der daraus resultierende Druck auf den Schaft als unangenehm empfunden, bzw. wird das Einsteigen in die Prothese erschwert. Da bei den meisten Prothesenträgern Stumpfvolumenschwankungen zu beobachten sind, ändert sich die resultierende Passung zwischen Aufnahmehülse und Liner.

Weiter kann bei Bewegung des Stumpfes, z. B. beim Gehen kurzzeitig ein Spalt zwischen dem Stumpf und der Innenseite der Aufnahmehülse entstehen durch welchen der Innendruck entweicht und sich der Sitz des Stumpfes in der Aufnahmehülse verschlechtert. Dies ist umso mehr der Fall, wenn aufgrund eines gewünschten Tragekomforts die Passung des Stumpfes gegenüber der Aufnahmehülse nicht mehr so ausgeprägt ist. Das Herausgleiten des Stumpfes aus der Stumpfaufnahmehülse in der Hebebewegung des Körpergliedes ist dabei oft die Folge.

In der EP 0 631 765 B1 wird zur Lösung dieses Problems eine Stumpfaufnahmevorrichtung vorgeschlagen, in welche ein Dichtungselement eingebracht wird, welches nahezu kreisförmig ausgebildet ist und mittig eine Öffnung aufweist, durch welche der Stumpf in die Aufnahmehülse eingebracht wird. Eine Abdichtung erfolgt dadurch stetig, auch bei Bewegung des Stumpfes in der Aufnahme zwischen dieser Dichtungsmembran und dem Liner bzw. Stumpf selbst.

Das beschriebene Dichtungselement ist umlaufend mit einem Ring versehen, welcher in eine in die Stumpfaufnahmehülse eingebrachte Nut eingreift und dort fixiert wird.

Als Nachteil dieser Ausführungsformen hat sich gezeigt, dass zum einen die Abdichtung in der Nut, in welche der Ring des Dichtungselementes eingreift, problematisch oder nur sehr aufwendig zu realisieren ist und zum anderen die Formschlüssigkeit des Ringes in der Nut oft nicht ausreichend ist.

Beim Einsteigen und Aussteigen mit dem Stumpf in oder aus der Stumpfaufnahmenhülse wird oft der umlaufende Ring aus der Nut herausgezogen, wodurch natürlich entweder die Funktion nicht gegeben ist oder das Dichtungselement mühselig wieder eingepasst werden muss. Weiter ist es sehr aufwändig, die umlaufende Nut so zu realisieren, dass diese formschlüssig und blasenfrei auf der Innenseite der Gliedmaßenstumpfaufnahmehülse ausgebildet ist.

Aufgabe der vorliegenden Erfindung ist es eine Gliedmaßenstumpfaufnahmehülse mit integrierter Aufnahme für ein Dichtelement zu schaffen, in welche der Stumpf eines Patienten einfach einzubringen ist und in welcher der Stumpf einen guten, sicheren und festen Sitz durch entstehenden Unterdruck im distalen Bereich der Stumpfaufnahmehülse aufweist.

Diese Aufgabe wird mit den Merkmalen des kennzeichnenden Teils des Anspruchs 1 gelöst. Fortbildungen und vorteilhafte Ausführungen der Erfindung sind in den weiteren Ansprüchen umfasst.

Erfindungsgemäß ist eine Gliedmaßenstumpfaufnahmehülse mit integrierbarer Aufnahme für ein nach Innen reichendes Dichtungselement, das reversibel, formschlüssig und von Luft nicht hinterströmbar anzubringen ist.

Dadurch wird die Arretierung eines Gliedmaßenstumpfes mittels Abdichtung eines distal befindlichen Bereiches im Inneren der Stumpfaufnahmehülse zum Gliedmaßenstumpf und der umgebenden Atmosphäre hin gewährleistet.

Die mit der erfindungsgemäßen Aufnahme und dem zugehörigen Dichtungselement ausgerüstete Stumpfaufnahmehülse verfügt bevorzugt auch über ein Ventil in ihrem distalen Bereich unter der Dichtungsvorrichtung. Beim Einsteigen mit dem Stumpf in die Aufnahmehülse wird der Stumpf durch das Dichtelement hindurch in die Hülse eingeführt und die dabei komprimierte Luft kann durch das Ventil nach außen entweichen. Durch die bevorzugte Ausführung des Ventils wird verhindert, dass, ohne Öffnen des Ventils Luft von Außen in den distalen Bereich der Stumpfaufnahmehülse nachströmen kann, der durch Zugkraft beim Versuch den Stumpf aus der Hülse heraus zu ziehende entstehende Unterdruck bleibt somit erhalten und fixiert den Stumpf in der Aufnahmehülse.

Das Funktionselement, an welches das Dichtungselement anbringbar ist, wird bevorzugt in eine, auf der Innenseite der Aufnahmehülse vorhandene, bevorzugt umlaufende Nut, unlösbar und von Luft nicht hinterströmbar, fixiert, wobei das Funktionselement dazu umschlossen, eingepresst, eingeklebt, eingegossen oder z.B. auch einvulkanisiert sein kann.

Das Funktionsteil ist bevorzugt in einem elastischen oder wenigstens teilelastischen Material ausgeführt und verfügt über gute Dichteigenschaften. Nach einer weiteren erfindungsgemäßen Ausführung besitzt das Funktionselement eine Hinterschneidung, bzw. läuft nach hinten trapezförmig verbreiternd zu, welche in eine entsprechende Nut in der Aufnahmehülse eingreift und wodurch ein fester Sitz in der Nut mit entsprechender Abdichtung erreicht wird.

Das in die Nut in der Stumpfaufnahmehülse fixierte Funktionselement, an welches ein nach Innen reichendes Dichtungselement mit einer bevorzugt mittigen, bevorzugten triovalen bzw. ovalen oder auch kreisförmigen Öffnung anbringbar ist, bildet somit eine im Inneren der Stumpfaufnahmehülse umlaufende ringförmige Aufnahme.

Diese Aufnahme besitzt bevorzugt auf ihrer, dem Inneren der Stumpfaufnahmenhülse zugewandten Seite, eine umlaufende Nut, in welche wiederrum das Dichtungselement reversibel, formschlüssig und von Luft nicht hinterströmbar, anbringbar ist.

In die beschriebene umlaufende Nut in dem Funktionselement wird das Dichtungselement mittels eines im wesentlichen starren, an die Stumpfform anpassbaren, umlaufenden Ring formschlüssig und von Luft nicht hinterströmbar angebracht.

Durch diese bevorzugte Ausführung entsteht eine dichtende Verbindung zwischen dem Funktionsteil und dem Dichtungselement, wobei sich das Dichtungselement beim Einsteigen mit dem Stumpf in die Aufnahmehülse, am Stumpf anlegt und diesen umlaufend gegenüber der äußeren Atmosphäre abdichtet. Der im distalen Bereich der Aufnahmehülse, unterhalb des Ringes des Funktionselements erzeugte Druck kann durch das Ventil entweichen und es entsteht dort der beim Herausziehen des Stumpfes beschriebene Unterdruck im Inneren der Aufnahmehülse, durch welchen der Stumpf fixiert wird.

Das Dichtungselement, welches sich um den Stumpf, bzw. Liner schmiegt, wird, beim Einstieg des Stumpfes, zusammen mit dem Stumpf in Richtung des distalen Endes der Stumpfhülle gezogen und legt sich dort zwischen dem Stumpf und der Innenwand der Stumpfhülse, umlaufend an. An der Innenseite der Aufnahmehülse ist distal des umlaufenden Funktionselements, eine Aussparung bzw. Verjüngung vorgesehen, in welche sich die Dichtmembran und insbesondere der Übergang zwischen Membran und Umlaufring nach dem Einsteigen zurückziehen kann, damit in diesem Bereich kein zusätzlich zirkular wirkender Druck auf den Stumpf aufgebaut wird, welcher physiologisch nachteilig wäre.

Auch das umlaufende Funktionselement selbst, in welches das Dichtungselement in der Nut fixiert ist, weist an seinem distalen Ende in der Nut der Stumpfaufnahmehülse eine Aussparung auf, ist also an dieser Stelle in der Nut im Durchmesser kleiner, um dem, sich beim Einsteigen des Amputierten verformende Dichtelement, in diesem Bereich Platz zu verschaffen.

Vor dem Einstieg eines Amputierten, mit seinem, üblicherweise mit einem Liner überzogenen Stumpf, ist die Stumpfaufnahmehülse mit dem Dichtungselement zu versehen, welches mit seinem umlaufenden, unlösbar mit der Dichtungsmembran verbundenen, härteren Rand in die Nut des im Inneren der Stumpfaufnahme fixierten Funktionselement formschlüssig verbunden.

Die Dichtungsmembran selbst ist bevorzugt aus einem Elastomermaterial ausgeführt und besitzt im wesentlichen mittig eine, im wesentlichen triovalen bzw. ovalen oder auch kreisrunde Öffnung, durch welche hindurch der Gliedmaßenstumpf in die Stumpfaufnahmehülse eingebracht wird. Die Materialien der Dichtungsmembran sind bevorzugt derart ausgeführt, dass eine Anhaftung des Dichtungsteils bzw. der Dichtungsmembran an verwendeten Linern unterbunden wird.

Der um die Dichtungsmembran verlaufende Spannring ist bezüglich Material und Umfang so ausgeführt, dass dieser sich durch Verdrehen einfach in die Hülse einbringen und in die Nut an der, der Innenseite der Aufnahmehülse zugewandten Seite des Funktionsteiles anbringen lässt und dort eine formschlüssige und dichtende Verbindung gegenüber dem umlaufenden Funktionselement-Ring bildet.

Diese Verbindung ist so formschlüssig, dass sie dem Zug auf das Dichtungselement beim Einbringen, sowie Herausführen des Stumpfes aus der Aufnahmehülse problemlos und ohne sich hinterströmen zu lassen standhält, womit eine unangenehme Bildung von Falten oder Überständen vermieden wird, welche im Bereich der Abdichtung gegen den Stumpf drücken könnten.

Die Fixierung des Stumpfes in der Gliedmaßenstumpfaufnahmehülse erfolgt, wie genannt, durch den entstehenden Unterdruck im distalen Bereich der Aufnahmehülse. Das beschriebene Volumenkompartement kann bevorzugt durch, ein im Schaft integriertes Ventil, wieder hinterlüftet werden um dem Patient zu ermöglichen, den Stumpf wieder aus der Aufnahmehülse heraus zu ziehen, wobei dazu sowohl mehrere Ventile zum Einsatz kommen können oder in verschiedene Strömungsrichtung sperrbare, bzw. richtungsändernde Ventile zum Einsatz kommen können.

Im Folgenden wird die Erfindung anhand von Zeichnungen beispielhaft näher beschrieben. Dabei zeigen:
- Fig. 1: eine Darstellung des Dichtelements angebracht in dem Funktionselement welches in die Aufnahmehülse eingebracht ist.
- Fig. 2: eine perspektivische Darstellung eines Funktionselements
- Fig. 3: eine perspektivische Darstellung eines Dichtelements
- Fig. 4: eine perspektivische Ansicht einer Ausführung eines Halterings
- Fig. 5: eine perspektivische Ansicht der Gesamtvorrichtung

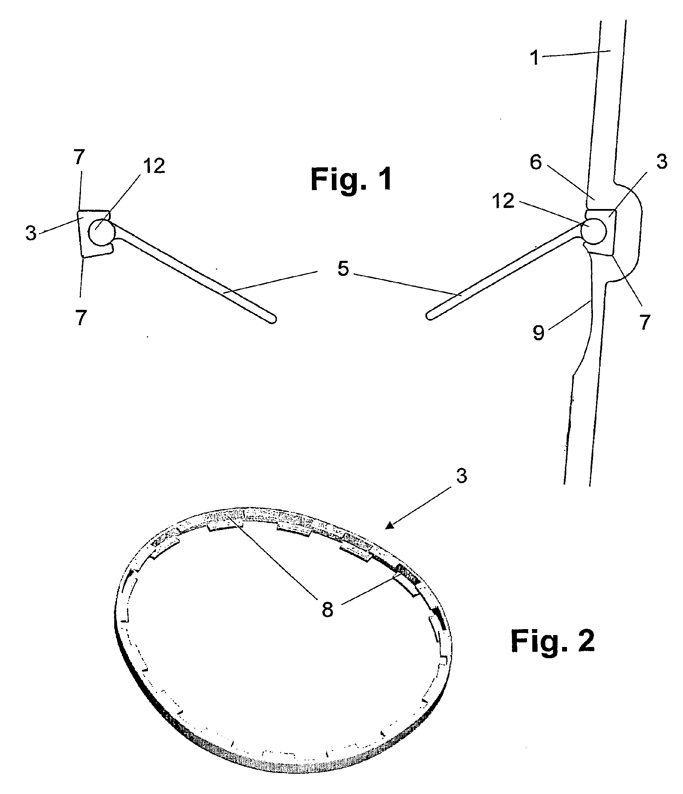

In Fig. 1 ist praktisch ein Ausschnitt aus einem Schnitt durch eine Aufnahmehülse 1 und durch eine komplette Vorrichtung 4 zu sehen, bei welchem auf der linken Seite jedoch die Aufnahmehülse weggelassen wurde. In der Aufnahmehülse 1 ist eine Nut 6 eingearbeitet, welche, wie zu sehen ist, mit einer Hinterschneidung versehen ist und in welche das Funktionselement 3 der Gesamtvorrichtung 4 bevorzugt dauerhaft eingebracht wird. Das Funktionselement 3 verfügt für einen besseren Sitz in der Nut 6 der Aufnahmehülse 1 über einen, der Hinterschneidung in der Aufnahmehülse 1 entsprechenden Schwalbenschwanz und ist darüberhinaus durch geeignete Maßnahmen wie einkleben oder eingießen dauerhaft und sicher fixiert.

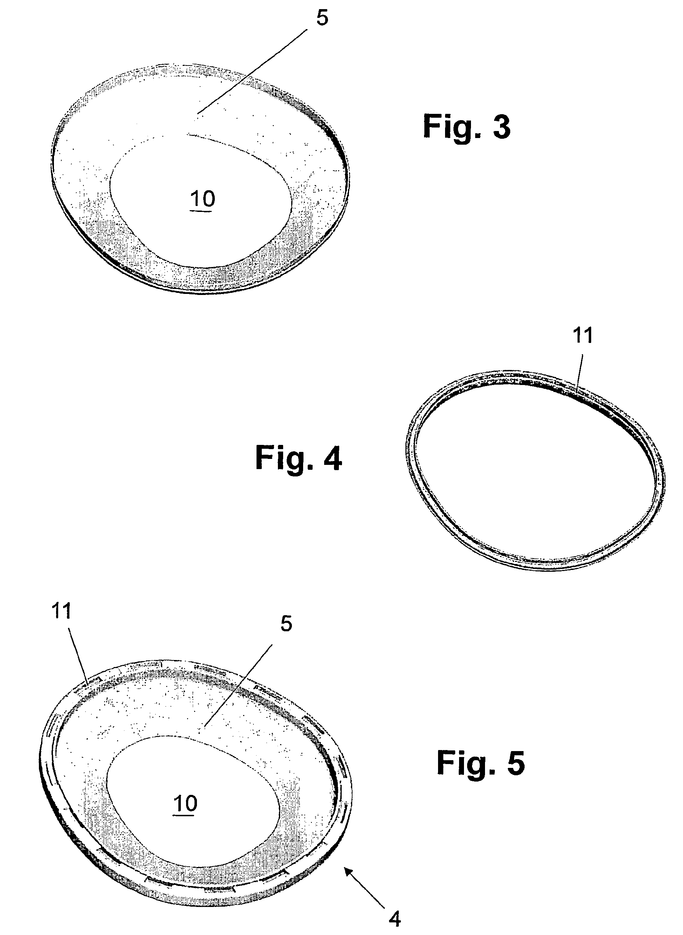

Das Funktionselement 3, welches in der Aufnahmehülse 1 umlaufend fixiert ist, verfügt seinerseits, wie in Fig. 2 gut zu erkennen ist, ebenfalls über eine umlaufende Nut 8, in welche das Dichtungselement 5, in Fig. 3 gezeigt anbringbar ist und welches in dieser Nut 8 formschlüssig und von Luft nicht hinterströmbar angebracht ist.

Diese Verbindung wird durch einen, am Dichtungselement 5 dauerhaft und unlösbar angebrachten Ring 12 oder mittels eines, aus härterem Material bestehenden, Haltering 11, wie in Fig. 4 gezeigt, erreicht.

In Fig. 5 ist die Vorrichtung, wie sie in der umlaufenden Nut 6 in der Aufnahmehülse endgültig zum Einsatz kommt, gezeigt. Das Funktionsteil 3 verbleibt nach der Erstmontage aber in der Nut 6 in der Aufnahmehülse 1, wo es dauerhaft fixiert ist und es kann lediglich das Dichtelement 5 und der Haltering 11 entfernt und ggf. ausgewechselt werden.

Der Einstieg des Amputierten mit seinem Stumpf in die Gliedmaßenaufnahmehülse 1 erfolgt wie bekannt, weitgehend mittig, hindurch durch die Öffnung 10 im Dichtungselement 5.

Dabei legt sich das Dichtungselement 5 an den Stumpf bzw. an den, den Stumpf umgebenden Liner an und dichtet diesen, bzw. den distalen Bereich der Aufnahmehülse zur Atmosphäre hin ab.

Die Wandstärke des Dichtungselementes 5, welche nun den Stumpfdurchmesser an dieser Stelle erhöht, kann sich beim weiteren Einführen des Stumpfes in die Hülse in eine Ausnehmung 9, in Fig. 1 gezeigt, an der Innenseite der Hülse 1 zurückziehen. Auch für den direkten Übergang zwischen Dichtungselement 5 und der Aufnahme 12 ist ein entsprechender Rückzugraum geschaffen, wodurch es bei der endgültigen Position des Stumpfes in der Aufnahmehülse keine Überstände des Dichtungselementes gibt. Durch diese Maßnahmen wird ein zusätzlicher Druck durch das Dichtungselement 5 oder dem Übergang auf den Stumpf effektiv vermieden und es kommt zu keinem zusätzlich zirkular wirkenden Druck auf den Stumpf, welcher physiologisch nachteilig wäre.

Nachdem bevorzugte Ausführungen der Erfindung in Bezug auf die beiliegenden Zeichnungen beschrieben wurden, ist festzuhalten, dass die Erfindung nicht auf diese genauen Ausführungen beschränkt ist und dass verschiedene Änderungen und Modifizierungen daran von einem Fachmann ausgeführt werden können, ohne dass vom Umfang der Erfindung, wie er in den beiliegenden Ansprüchen definiert ist abgewichen wird.

## Patentansprüche

1. Gliedmaßenstumpfaufnahmehülse (1) mit integrierbarer Arretiervorrichtung (4) zur Arretierung eines Gliedmaßenstumpfes mittels Abdichtung eines distal befindlichen Bereiches des Inneren der Gliedmaßenstumpfaufnahmehülse (1) zum Gliedmaßenstumpf und der umgebenden Atmosphäre hin, wobei in die Innenseite der Gliedmaßenstumpfaufnahmehülse (1) ein Funktionselement (3) der Arretiervorrichtung (4) integriert ist, an welches ein nach innen reichendes Dichtungselement (5) reversibel formschlüssig und von Luft nicht hinterströmbar anbringbar ist, **dadurch gekennzeichnet, dass** das Funktionselement (3) zur Anbringung des Dichtungselements (5) in einer Nut (6) der Gliedmaßenstumpfaufnahmehülse (1) unlösbar und von Luft nicht hinterströmbar fixiert ist.

2. Gliedmaßenstumpfaufnahmehülse mit integrierbarer Arretiervorrichtung nach Anspruch 1, **dadurch gekennzeichnet, dass** das Funktionselement (3) zur Einbringung des Dichtungselements (5) mit einer Hinterschneidung (7) zur formschlüssigen Integration in eine entsprechende Nut (6) der Innenseite der Gliedmaßenstumpfaufnahmehülse (1) eingebracht ist.

3. Gliedmaßenstumpfaufnahmehülse mit integrierbarer Arretiervorrichtung nach Anspruch 1 oder 2, **dadurch gekennzeichnet, dass** das Funktionselement (3) zur Einbringung des Dichtungselements (5) auf seiner dem Inneren der Gliedmaßenstumpfaufnahmehülse (1) zugewandten Seite umlaufend eine Nut (8) aufweist, in welche das Dichtungselement (5) reversibel, formschlüssig und von Luft nicht hinterströmbar anbringbar ist.

4. Gliedmaßenstumpfaufnahmehülse mit integrierbarer Arretiervorrichtung nach Anspruch 3, **dadurch gekennzeichnet, dass** das Dichtungselement (5) aus einem Elastomermaterial und einem umlaufend um das Elastomermaterial angeordneten, mit diesem verbundenen härteren Material (11) besteht, mittels welchem das Dichtungselement (5) an die dem Inneren der Gliedmaßenstumpfaufnahmehülse (1) zugewandte Nut (8) des Funktionselements (3) reversibel formschlüssig und von Luft nicht hinterströmbar anbringbar ist.

5. Gliedmaßenstumpfaufnahmehülse mit integrierter Arretiervorrichtung nach einem der vorangehenden Ansprüche, **dadurch gekennzeichnet, dass** die Gliedmaßenstumpfaufnahmehülse (1) eine distal des Funktionselements (3) zur Anbringung des Dichtelements (5) befindliche Ausnehmung (9) aufweist.

6. Gliedmaßenstumpfaufnahmehülse mit integrierbarer Arretiervorrichtung nach einem der vorangehenden Ansprüche, **dadurch gekennzeichnet, dass** das Dichtungselement (5) eine seine Mitte umgebende Öffnung (10) aufweist, durch die der Gliedmaßenstumpf in die Gliedmaßenstumpfaufnahmehülse (1) einbringbar ist.

7. Gliedmaßenstumpfaufnahmehülse mit integrierbarer Arretiervorrichtung nach einem der vorangehenden Ansprüche, **dadurch gekennzeichnet, dass** die Gliedmaßenstumpfaufnahmehülse (1) ein Ventil aufweist, welches die beim Einbringen des Gliedmaßenstumpfes in die Gliedmaßenstumpfaufnahmehülse (1) zwischen Gliedmaßenstumpf, Dichtungselement (5) und Gliedmaßenstumpfaufnahmehülse (1) befindliche Luft abströmen lässt, ohne dass im Falle eines dort befindlichen Unterdrucks Luft in dieses Volumenkompartement einströmt.

8. Gliedmaßenstumpfaufnahmehülse mit integrierbarer Arretiervorrichtung nach Anspruch 7, **dadurch gekennzeichnet, dass** sie ein in den Schaft zu integrierendes Ventil aufweist, mit dem das Volumenkompartement hinterlüftet werden kann, um dem Amputierten zu ermöglichen, den Gliedmaßenstumpf wieder aus der Gliedmaßenstumpfaufnahmehülse (1) zu ziehen.

9. Gliedmaßenstumpfaufnahmehülse mit integrierbarer Arretiervorrichtung nach einem der vorangehenden Ansprüche, **dadurch gekennzeichnet, dass** das elastische Dichtungselement (5) gegenüber der Lineroberfläche um den Gliedmaßenstumpf eine nicht anhaftende Gleiteigenschaft aufweist.

## Claims

1. A limb-stump retaining sleeve (1) with an integrated locking device (4) for locking a limb-stump by sealing a distally disposed region of the interior of the limb-stump retaining sleeve (1) relative to the limb-stump and the surrounding atmosphere,
wherein a functional element (3) of the locking device (4), to which an inwardly extending sealing element (5) can be attached in a reversible, form-fit manner precluding a back-flow of air, is integrated into the interior of the limb-stump retaining sleeve (1),
**characterised in that**
the functional element for the attachment of the sealing element (5) is fixed, in a non-detachable manner precluding a back-flow of air, within a groove (6) of the limb-stump retaining sleeve (1).

2. The limb-stump retaining sleeve with integrated locking device according to claim 1,
**characterised in that**,
for the introduction of the sealing element (5), the functional element (3) is introduced with an undercut (7) for the form-fit integration into a corresponding groove (6) in the interior of the limb-stump retaining sleeve (1).

3. The limb-stump retaining sleeve with integrated locking device according to claim 1 or 2,
**characterised in that**,
for the introduction of the sealing element (5), the functional element (3) provides on its side facing towards the interior of the limb-stump retaining sleeve (1), a peripheral groove (8), in which the sealing element (5) can be attached in a reversible, form-fit manner precluding a back-flow of air.

4. The limb-stump retaining sleeve with integrated locking device according to claim 3,
**characterised in that**
the sealing element (5) comprises an elastomer material and a relatively harder material (11) connected to the latter arranged peripherally around the elastomer material, by means of which the sealing element (5) can be attached to the groove (8) facing towards the interior of the limb-stump retaining sleeve (1) of the functional element (3) in a reversible, form-fit manner precluding a back-flow of air.

5. The limb-stump retaining sleeve with integrated locking device according to any one of the preceding claims,
**characterised in that**
the limb-stump retaining sleeve (1) provides a recess (9) disposed distally to the functional element (3) for the attachment of the sealing element (5).

6. The limb-stump retaining sleeve with integrated locking device according to any one of the preceding claims,
**characterised in that**
the sealing element (5) provides an opening (10) around its centre, through which the limb-stump can be introduced into the limb-stump retaining sleeve (1).

7. The limb-stump retaining sleeve with integrated locking device according to any one of the preceding claims,
**characterised in that**
the limb-stump retaining sleeve (1) provides a valve, which allows the air disposed between the limb-stump, sealing element (5) and limb-stump retaining sleeve (1) to escape upon the introduction of the limb-stump into the limb-stump retaining sleeve (1) without air flowing into the this volume compartment in the presence of a vacuum pressure disposed there.

8. The limb-stump retaining sleeve with integrated locking device according to claim 7,
**characterised in that**
it provides a valve to be integrated into the shaft, with which the volume compartment can be back-ventilated, in order to allow the amputee to withdraw the limb-stump from the limb-stump retaining sleeve (1) again.

9. The limb-stump retaining sleeve with integrated locking device according to any one of the preceding claims,
**characterised in that**,
by comparison with the liner surface around the limb-stump, the elastic sealing element (5) provides an non-sticking sliding property.

## Revendications

1. Manchon de logement de moignon de membre (1) avec dispositif de blocage intégrable (4) pour le blocage d'un moignon de membre grâce à l'étanchéisation d'une zone distale de l'intérieur du manchon de logement de moignon (1) par rapport au moignon de membre et à l'atmosphère ambiante, moyennant quoi, à l'intérieur du manchon de logement de moignon de membre (1), est intégré un élément fonctionnel (3) du dispositif de blocage (4), sur lequel peut être fixé un élément d'étanchéité (5) dépassant vers l'intérieur, de manière réversible, à complémentarité de forme et non pénétrable par l'air, **caractérisé en ce que** l'élément fonctionnel (3) est fixé de manière inamovible et non pénétrable par l'air pour l'insertion de l'élément d'étanchéité (5) dans une rainure (6) du manchon de logement de moignon de membre (1).

2. Manchon de logement de moignon de membre avec dispositif de blocage intégrable selon la revendication 1, **caractérisé en ce que** l'élément fonctionnel (3) pour l'insertion de l'élément d'étanchéité (5) est inséré dans une rainure correspondante (6) de l'intérieur du manchon de logement de moignon de membre (1) avec une contre-dépouille (7) pour une intégration à complémentarité de forme.

3. Manchon de logement de moignon de membre avec dispositif de blocage intégrable selon la revendication 1 ou 2, **caractérisé en ce que** l'élément fonctionnel (3) pour l'insertion de l'élément d'étanchéité présente, sur sa face orientée vers l'intérieur du manchon de logement de moignon de membre (1), une rainure circulaire (8) dans laquelle l'élément d'étanchéité (5) peut être inséré de manière réversible, à complémentarité de forme et non pénétrable par l'air.

4. Manchon de logement de moignon de membre avec dispositif de blocage intégrable selon la revendication 3, **caractérisé en ce que** l'élément d'étanchéité (5) est constitué d'un matériau élastomère et d'un matériau plus dur (11) disposé autour du matériau élastomère et relié à celui, à l'aide duquel l'élément d'étanchéité (5) peut être inséré dans la rainure (8), orientée vers l'intérieur du manchon de logement de moignon de membre (1), de l'élément fonctionnel (3) de manière réversible, à complémentarité de forme et non pénétrable par l'air.

5. Manchon de logement de moignon de membre avec dispositif de blocage intégré selon l'une des revendications précédentes, **caractérisé en ce que** le manchon de logement de moignon de membre (1) comprend un évidement (9) se trouvant à l'extrémité distal de l'élément fonctionnel (3) pour l'insertion de l'élément d'étanchéité (5).

6. Manchon de logement de moignon de membre avec dispositif de blocage intégrable selon l'une des revendications précédentes, **caractérisé en ce que** l'élément d'étanchéité (5) comprend une ouverture (10) entourant son centre, à travers laquelle le moignon de membre peut être inséré dans le manchon de logement de moignon de membre (1).

7. Manchon de logement de moignon de membre avec dispositif de blocage intégrable selon l'une des revendications précédentes, **caractérisé en ce que** le manchon de logement de moignon de membre (1) comprend une soupape qui, lors de l'insertion du moignon de membre dans le manchon de logement de moignon de membre (1), laisse s'écouler l'air se trouvant entre le moignon de membre, l'élément d'étanchéité (5) et le manchon de logement de moignon de membre (1), sans que de l'air s'écoule dans ce compartiment volumique dans le cas d'une dépression.

8. Manchon de logement de moignon de membre avec dispositif de blocage intégrable selon la revendication 7, **caractérisé en ce qu'**il comprend une soupape à intégrer dans la tige, avec laquelle le compartiment volumique peut être ventilé par l'arrière, afin de permettre à la personne amputée de retirer le moignon de membre hors du manchon de logement de moignon de membre (1).

9. Manchon de logement de moignon de membre avec dispositif de blocage intégrable selon l'une des revendications précédentes, **caractérisé en ce que** l'élément d'étanchéité élastique (5) présente, par rapport à la surface du liner, autour du moignon de membre, une propriété de glissement non adhésive.
